# EUROPEAN PATENT APPLICATION

(11) **EP 3 701 951 A1**
(43) Date of publication of application: **02.09.2020**
(21) Application number: 18869512.6
(22) Date of filing: 22.10.2018
(51) Int. Cl.: A61K 31/567, A61P 5/36, A61P 15/00

(54) **CRYSTAL FORM OF SELECTIVE PROGESTERONE RECEPTOR MODULATOR AND PREPARATION METHOD THEREFOR**

(30) Priority: 26.10.2017 CN 201711022041; 26.01.2018 CN 201810078734
(71) Applicant: Crystal Pharmaceutical (Suzhou) Co., Ltd., Suzhou, Jiangsu 215123 (CN)
(72) Inventor: CHEN, Minhua, Suzhou Jiangsu 215123 (CN); ZHANG, Yanfeng, Suzhou Jiangsu 215123 (CN); ZHANG, Jing, Suzhou Jiangsu 215123 (CN); ZHANG, Xiaoyu, Suzhou Jiangsu 215123 (CN); LIU, Kai, Suzhou Jiangsu 215123 (CN)
(74) Representative: Grünecker Patent- und Rechtsanwälte PartG mbB
(86) International application number: PCT/CN2018/111270
(87) International publication number: WO 2019/080811

(57) **Abstract**

The present disclosure relates to novel crystalline forms of compound I and processes for preparation thereof. The present disclosure also relates to pharmaceutical composition containing crystalline forms, and use of crystalline forms for preparing drugs containing selective progesterone receptor modulator, and use of crystalline forms for preparing drugs treating uterine fibroids and/or endometriosis. The crystalline forms of the present disclosure have one or more improved properties compared with prior art and have significant values for future drug optimization and development.

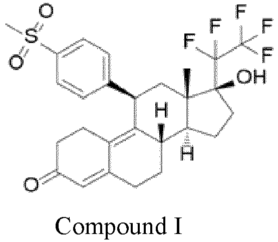

## Description

### TECHNICAL FIELD

The present disclosure relates to the field of pharmaceutical chemistry, particularly relates to crystalline forms of selective progesterone receptor modulator and processes for preparation thereof.

### BACKGROUND

Uterine fibroids are the most common benign tumors in women, and the recurrence rate of uterine fibroids after myomectomy is approximately 40%. Endometriosis is a common disease in women during their reproductive years, and the incidence of endometriosis in women is increasing year by year. At present, effective drugs can only be used for short-term use and treatment, while long-term use will lead to systemic adverse reactions. Selective progesterone receptor modulators are new progesterone receptor ligands. When bound to progesterone receptors, selective progesterone receptor modulators exhibit partial agonistic and antagonistic activity at progesterone in vivo. Selective progesterone receptor modulators have high receptors and target specificity, can effectively relieve the clinical symptoms of uterine fibroids, and shrink the fibroids, without affecting ovarian secretion of estrogen. Selective progesterone receptor modulators have no androgenic activity, so long-term use won't lead to adverse reactions such as development of male characteristics, osteoporosis and cardiovascular disease.

BAY-1002670 (Vilaprisan), developed by Bayer, is a small molecule selective progesterone receptor modulator. BAY-1002670 is expected to be the first drug that can be used for long-term oral treatment of uterine fibroids. In addition, studies have shown that BAY-1002670 can be a candidate drug for the treatment of endometriosis.

The chemical name of BAY-1002670 is (11β, 17β)-17-hydroxy-11-[4-(methylsulfonyl)phenyl]-17-(pentafluoroethyl)estra-4,9-diene-3-one (hereinafter referred to as "Compound I"), and the structure is shown as follows:

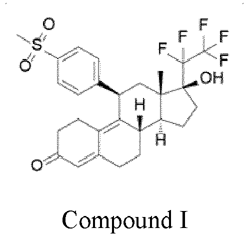

Crystalline forms are different solid forms formed by different arrangements of the compound molecules in crystal lattices. Polymorphism is the ability of a compound to exist in more than one crystalline form.

Crystalline form difference can affect drug's in vivo dissolution and absorption, which will further affect drug's clinical efficacy and safety to some extent. Especially for poorly soluble solid or semisolid oral drugs, the above effects of the crystalline form will be greater. Therefore, in the development of solid oral formulations, researches on crystalline forms are beneficial to selection of clinically meaningful, stable and controllable crystalline form. Drug polymorphism is an important part of drug research, test and regulation, and is also an important part of drug quality control.

The prior art CN102482317A disclosed a preparation method of BAY-1002670, and the inventors of the present disclosure obtained amorphous BAY-1002670 according to the preparation method of the compound I disclosed in CN102482317A. Molecules are randomly arranged in amorphous solids, so amorphous solids are in a thermodynamically unstable state. The inventors found that the amorphous BAY-1002670 is unstable under the conditions of 25 °C/60% RH (Relative Humidity), 40 °C/75% RH and 60 °C/75% RH. In addition, the preparation of amorphous is usually a rapid precipitation process to produce kinetically stable solid. The results show that residual solvent of amorphous is significantly higher than specification and the particle property control in the preparation process is difficult, making it a challenge in the application of the amorphous. Therefore, it is necessary to look for crystalline forms of BAY-1002670 for drug development. At present, no patent or literature has disclosed the crystalline form of BAY-1002670.

In order to overcome the disadvantages of prior art, the inventors of the present disclosure surprisingly discovered crystalline form CS2 and crystalline form CS4 of BAY-1002670, which have advantages in at least one aspect of stability, melting point, solubility, in vitro and in vivo dissolution, hygroscopicity, bioavailability, adhesiveness, compressibility, flowability, processability, purification ability, formulation production, etc. Particularly, they don't degrade easily and have advantages of good physical and chemical stability, small particle size, uniform particle size distribution, low hygroscopicity and low solvent residue, which provides a new and better choice for the development of BAY-1002670 and are of great significance.

### SUMMARY

The main objective of the present disclosure is to provide crystalline forms of BAY-1002670, processes for preparation and use thereof.

According to the objective of the present disclosure, crystalline form CS2 of BAY-1002670 is provided (hereinafter referred to as Form CS2).

According to one aspect of the present disclosure, the X-ray powder diffraction pattern of Form CS2 shows characteristic peaks at 2theta values of 4.0°±0.2°, 15.9°±0.2° and 17.9°±0.2° using CuKa radiation.

Furthermore, the X-ray powder diffraction pattern of Form CS2 shows one or two or three characteristic peaks at 2theta values of 19.0°±0.2°, 20.4°±0.2° and 21.4°±0.2°. Preferably, the X-ray powder diffraction pattern of Form CS2 shows three characteristic peaks at 2theta values of 19.0°±0.2°, 20.4°±0.2° and 21.4°±0.2°.

Furthermore, the X-ray powder diffraction pattern of Form CS2 shows one or two or three characteristic peaks at 2theta values of 11.8°±0.2°, 15.0°±0.2° and 25.1°±0.2°. Preferably, the X-ray powder diffraction pattern of Form CS2 shows three characteristic peaks at 2theta values of 11.8°±0.2°, 15.0°±0.2° and 25.1°±0.2°.

According to another aspect of the present disclosure, the X-ray powder diffraction pattern of Form CS2 shows three or four or five or six or seven or eight or nine or ten or eleven or twelve or thirteen characteristic peaks at 2theta values of 4.0°±0.2°, 15.9°±0.2°, 17.9°±0.2°, 19.0°±0.2°, 20.4°±0.2°, 21.4°±0.2°, 11.8°±0.2°, 15.0°±0.2°, 25.1°±0.2°, 14.5°±0.2°, 16.9°±0.2°, 17.3°±0.2° and 18.5°±0.2°.

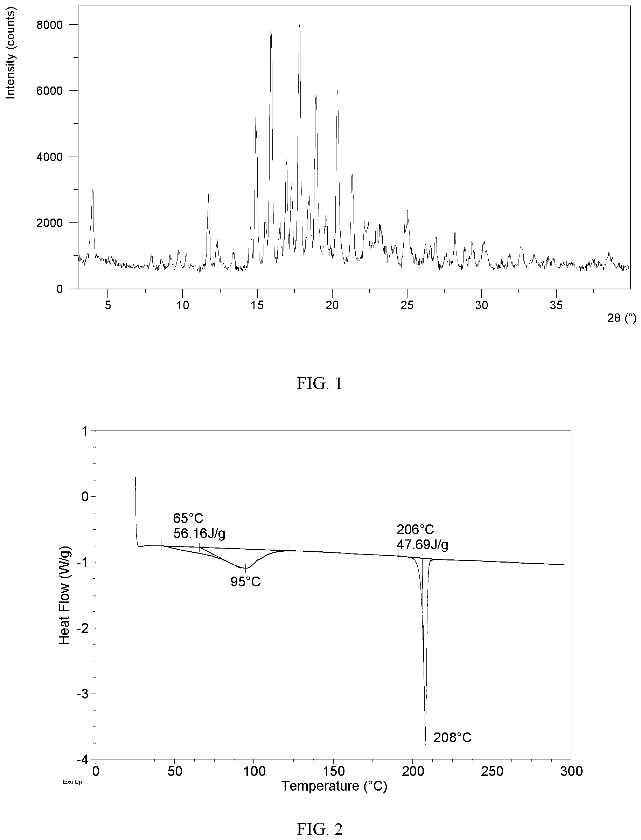

Without any limitation being implied, in a specific embodiment, the X-ray powder diffraction pattern of Form CS2 is substantially as depicted in Figure 1.
Without any limitation being implied, in a specific embodiment, the unit cell parameters of Form CS2 are substantially as depicted in the following table.

| Crystal system | monoclinic | | | | |
|---|---|---|---|---|---|
| Space group | *C2* | *a* | 21.432(2) Å | *α* | 90° |
| | | *b* | 10.7076(10) Å | *β* | 98.346(3)° |
| | | *c* | 22.438(2) Å | *γ* | 90° |
| Volume of unit cell (V) | 5094.4(8) A³ | | | | |
| Number of formula units in unit cell (Z) | 8 | | | | |
| Calculated density | 1.464 g/cm³ | | | | |

According to the objective of the present disclosure, a process for preparing Form CS2 is also provided, wherein the process comprises:
(1) Adding BAY-1002670 into a mixture of ketones and water, and stirring to obtain crystalline form CS2 ; or
(2) Dissolving BAY-1002670 in alcohols, esters, or ketones, putting the solution in a closed system with water vapor to obtain crystalline form CS2 by liquid vapor diffusion; or
(3) Dissolving BAY-1002670 in ketones, putting the solution in a closed system with n-heptane vapor to obtain crystalline form CS2 by liquid vapor diffusion.

Furthermore, in method (1) said ketone is preferably acetone.

Furthermore, in method (2) said alcohol is preferably methanol, said ester is preferably ethyl acetate, said ketone is preferably butanone.

Furthermore, in method (3) said ketone is preferably methyl isobutyl ketone.

According to the objective of the present disclosure, crystalline form CS4 of BAY-1002670 is provided (hereinafter referred to as Form CS4).

According to one aspect of the present disclosure, the X-ray powder diffraction pattern of Form CS4 shows characteristic peaks at 2theta values of 16.0°±0.2°, 16.6°±0.2° and 14.1°±0.2° using CuKa radiation.

Furthermore, the X-ray powder diffraction pattern of Form CS4 shows one or two or three characteristic peaks at 2theta values of 18.9°±0.2°, 21.5°±0.2° and 10.1°±0.2°. Preferably, the X-ray powder diffraction pattern of Form CS4 shows three characteristic peaks at 2theta values of 18.9°±0.2°, 21.5°±0.2° and 10.1°±0.2°.

Furthermore, the X-ray powder diffraction pattern of Form CS4 shows one or two or three characteristic peaks at 2theta values of 13.2°±0.2°, 23.5°±0.2° and 17.9°±0.2°. Preferably, the X-ray powder diffraction pattern of Form CS4 shows three characteristic peaks at 2theta values of 13.2°±0.2°, 23.5°±0.2° and 17.9°±0.2°.

According to another aspect of the present disclosure, the X-ray powder diffraction pattern of Form CS4 shows three or four or five or six or seven or eight or nine or ten or eleven or twelve or thirteen characteristic peaks at 2theta values of 16.0°±0.2°, 16.6°±0.2°, 14.1°±0.2°, 18.9°±0.2°, 21.5°±0.2°, 10.1°±0.2°, 13.2°±0.2°, 23.5°±0.2°, 17.9°±0.2°, 19.6°±0.2°, 19.8°±0.2°, 20.2°±0.2° and 20.6°±0.2°.

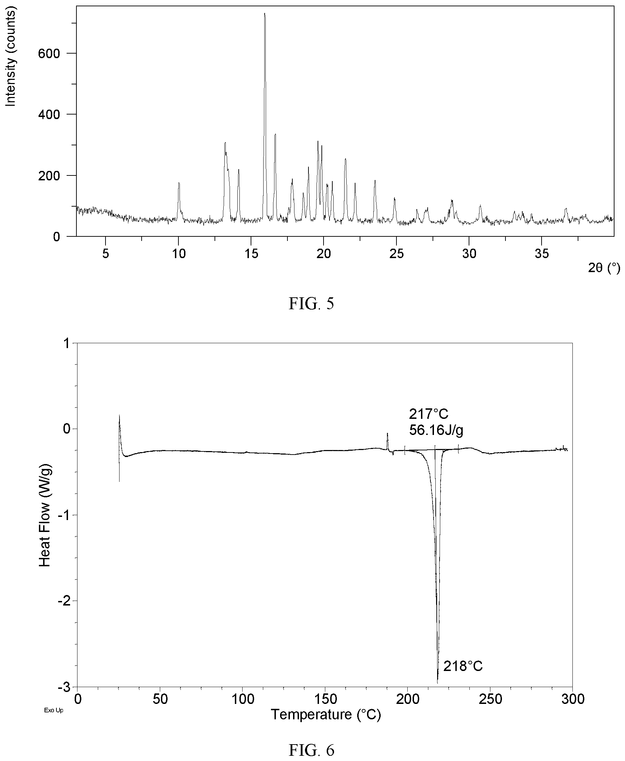

Without any limitation being implied, in a specific embodiment, the X-ray powder diffraction pattern of Form CS4 is substantially as depicted in Figure 5. According to the objective of the present disclosure, a process for preparing Form CS4 is also provided, wherein the process comprises:
(1) Adding BAY-1002670 into ethers, and stirring at 40 °C - 60 °C to obtain crystalline form CS4; or
(2) Dissolving BAY-1002670 in 2-methyltetrahydrofuran, evaporating to obtain a solid, and heating the solid to obtain crystalline form CS4; or
(3) Adding BAY-1002670 in a closed system with alcohol or ester vapor to obtain crystalline form CS4 by solid vapor diffusion.

Furthermore, in method (1) said ether is preferably methyl tert-butyl ether, said stirring temperature is preferably 50 °C.

Furthermore, in method (2) said heating temperature is 150 °C-200 °C, preferably 160 °C.

Furthermore, in method (3) said alcohol is preferably ethanol, said ester is preferably isopropyl acetate.

Form CS2 and Form CS4 of the present disclosure has the following advantages:
(1) Compared with prior art, crystalline forms of the present disclosure have lower hygroscopicity. The test results show that hygroscopicity of Form CS2 is less than 15% of that of the prior art solid, and hygroscopicity of Form CS4 is less than 10% of that of the prior art solid. The weight gain of Form CS2 and Form CS4 at 80% RH is 0.23% and 0.12%, respectively, while the weight gain of the prior art solids is up to 1.89%.
   Hygroscopicity affects the stability of drug substances, flowability and uniformity during the formulation process, thus affecting the quality of drug products. Hygroscopicity also affects the preparation, storage and post-treatment of drugs. The crystalline form with low hygroscopicity is not demanding on storage conditions, which reduces the cost of storage and quality control, and has strong economic value.
(2) Compared with prior art, crystalline forms of the present disclosure have good stability. Form CS2 and Form CS4 of the present disclosure remain unchanged for at least 6 months when stored under the conditions of 25 °C/60% RH and 40 °C/75% RH. Form CS2 and Form CS4 remain unchanged for at least 2 weeks when stored under the condition of 60 °C/75% RH. While the prior art solid partially converts to Form CS2 after being stored under the conditions of 40 °C/75% RH for 6 months or 60 °C/75% RH for 2 weeks.
   Good stability is of great importance to the drug development. From drug substance to drug product there are storage, transportation and formulation processes. The processes are often under stress conditions, which caused by the collision of drug substance in storage and transportation, the wet granulation process in drug production, the seasonal and regional climate differences, and weather factors. High temperature and high humidity is the most common stress condition. The prior art solid partially converted to a crystalline form after being stored under high temperature and high humidity conditions, while the crystalline forms of the present disclosure didn't changed.
   Crystalline form transformation can lead to changes in the absorption of the drug, and cause toxicity and side effects. The Form CS2 and Form CS4 have good physical stability, ensuring consistent and controllable quality of the drug substance and drug product, minimizing toxicity increase of the drug due to crystal transformation, and ensuring the therapeutic effect of the drug.
(3) Compared with prior art, crystalline forms of the present disclosure have good chemical stability. When stored under the condition of 25 °C/60% RH for 6 months, the purity of Form CS2 and Form CS4 of the present disclosure is only reduced by 0.06% and 0.13%, respectively, and remains substantially unchanged, while the purity of the prior art amorphous is reduced by 0.80%. The Form CS2 and Form CS4 of the present disclosure remain substantially unchanged under the condition of 40 °C/75% RH for 3 months, and the purity of Form CS2 and Form CS4 is only reduced by 0.27% and 0.02%, respectively, while the purity of the prior art amorphous is reduced by 1.08%. The purity of the Form CS2 and Form CS4 of the present disclosure substantially remain unchanged when stored under the condition of 60 °C/75% RH for 2 weeks, and the purity of Form CS2 and Form CS4 is only reduced by 0.05% and 0.03%, respectively, while the purity of the prior art amorphous is reduced by 0.98%.
   Chemical purity is of great significance for ensuring drug efficacy, safety and preventing the occurrence of adverse effects. Impurities in drug products are the main factors affecting purity. If the drug contains impurities higher than limit, its physicochemical properties and drug appearance may change, and the stability will be affected. The increase in impurities will lead to significantly lowered active ingredient content or reduced drug activity, and will also lead to significantly increased toxicity and side effects of the drug products. Compared with prior art, Form CS2 and Form CS4 of the present disclosure have little change in purity after storage, and are non-degradable. The purity remains substantially unchanged during storage, which effectively overcome the disadvantages of reduction in drug purity, poor efficacy and increased toxicity.
(4) Form CS2 and Form CS4 of the present disclosure have almost no residual solvent and meet the requirements of drug substance, while the residual solvent of the prior art exceeds the standard and cannot be used as a drug substance directly. Many organic solvents are harmful to human and environment. Therefore, in order to ensure drug safety and product quality, it is necessary to control the residual organic solvent of drug substance.

Furthermore, Form CS2 and Form CS4 of the present disclosure also have the following advantages:
Form CS2 and Form CS4 of the present disclosure have uniform particle size distribution. Their uniform particle size helps to ensure uniformity of drug substance content and dissolution in vitro. At the same time, the preparation process can be simplified, the pretreatment of the drug substance is not required, the cost is reduced, and the risk of decrease in crystallinity and crystal transformation caused by grinding can be reduced. According to the objective of the present disclosure, a pharmaceutical composition is provided. Said pharmaceutical composition comprises a therapeutically and/or prophylactically of Form CS2 or Form CS4 or combinations thereof and pharmaceutically acceptable carriers, diluents or excipients.

Furthermore, Form CS2 or Form CS4 or combinations thereof can be used for preparing selective progesterone receptor modulator drugs.

Furthermore, Form CS2 or Form CS4 or combinations thereof can be used for preparing drugs treating uterine fibroids and/or endometriosis.

In the present disclosure, said "liquid vapor diffusion" method for crystallization generally means that a solid is dissolved in solvents, and the solution is put into a closed system with anti-solvent vapor to obtain crystalline forms by vapor diffusion.

Said "solid vapor diffusion" method for crystallization generally means that a solid is put in a closed system with solvent vapor to obtain crystalline forms by vapor diffusion.

In the present disclosure, "crystal" or "crystalline form" refers to the crystal or the crystalline form being identified by the X-ray diffraction pattern shown herein. Those skilled in the art are able to understand that physicochemical properties discussed herein can be characterized. The experimental errors depend on the instrument conditions, the sampling processes and the purity of samples. In particular, those skilled in the art generally know that the X-ray diffraction pattern typically varies with the experimental conditions. It is necessary to point out that, the relative intensity of the diffraction peaks in the X-ray diffraction pattern may also vary with the experimental conditions; therefore, the order of the diffraction peak intensities cannot be regarded as the sole or decisive factor. In fact, the relative intensity of the diffraction peaks in the X-ray powder diffraction pattern is related to the preferred orientation of the crystals, and the diffraction peak intensities shown herein are illustrative and identical diffraction peak intensities are not required. In addition, the experimental error of the diffraction peak position is usually 5% or less, and the error of these positions should also be taken into account. An error of ±0.2° is usually allowed. In addition, due to experimental factors such as sample thickness, the overall offset of the diffraction peak is caused, and a certain offset is usually allowed. Thus, it will be understood by those skilled in the art that a crystalline form of the present disclosure is not necessarily to have the exactly same X-ray diffraction pattern of the example shown herein. As used herein, "the same XRPD pattern" does not mean absolutely the same, the same peak positions may differ by ±0.2° and the peak intensity allows for some variability. Any crystalline forms whose X-ray diffraction patterns have the same or similar characteristic peaks should be within the scope of the present disclosure. Those skilled in the art can compare the patterns shown in the present disclosure with that of an unknown crystalline form in order to identify whether these two groups of patterns reflect the same or different crystalline forms.

In some embodiments, Form CS2 and Form CS4 of the present disclosure are pure and substantially free of any other crystalline forms. In the present disclosure, the term "substantially free" when used to describe a novel crystalline form, it means that the content of other crystalline forms in the novel crystalline form is less than 20% (w/w), specifically less than 10% (w/w), more specifically less than 5% (w/w) and further more specifically less than 1% (w/w).

It should be noted that the number and the number range should not be understood as the number or number range themselves only. It should be understood by those skilled in the art that the specific number can be shifted at specific technical environment without departing from the spirit and principle of the present disclosure. In the present disclosure, the shift ranges expected by those skilled in the art is represented by the term "about".

### BRIEF DESCRIPTION OF THE DRAWINGS

Figure 1 shows an XRPD pattern of Form CS2 in Example 1.
Figure 2 shows a DSC curve of Form CS2 in Example 1.
Figure 3 shows a TGA curve of Form CS2 in Example 1.
Figure 4 shows an XRPD pattern of Form CS2 in Example 2.
Figure 5 shows an XRPD pattern of Form CS4 in Example 6.
Figure 6 shows a DSC curve of Form CS4 in Example 6.
Figure 7 shows a TGA curve of Form CS4 in Example 6.
Figure 8 shows an XRPD pattern of Form CS4 in Example 7.
Figure 9 shows an XRPD pattern of Form CS4 in Example 8.
Figure 10 shows an XRPD pattern overlay of Form CS2 before and after stored at 25 °C/60% RH for 6 months (top: before storage, bottom: after storage).
Figure 11 shows an XRPD pattern overlay of Form CS4 before and after stored at 25 °C/60% RH for 6 months (top: before storage, bottom: after storage).

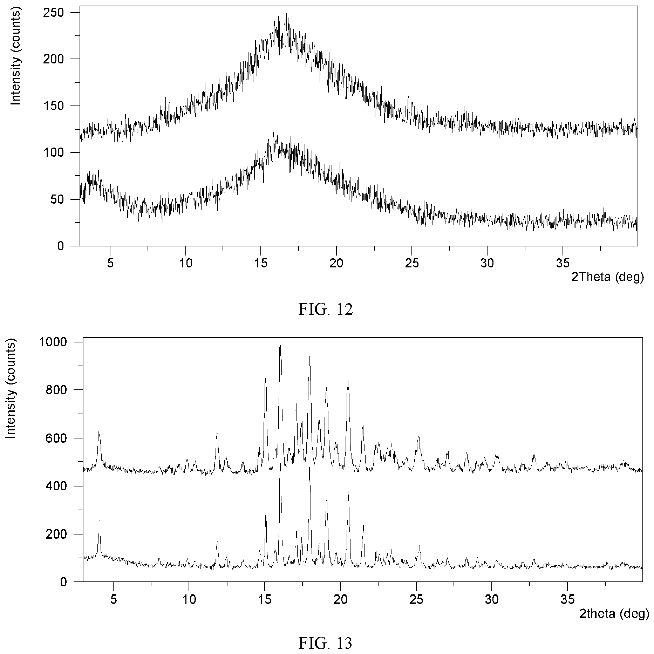

Figure 12 shows an XRPD pattern overlay of the prior art solid before and after stored at 25 °C/60% RH for 6 months (top: before storage, bottom: after storage).
Figure 13 shows an XRPD pattern overlay of Form CS2 before and after stored at 40 °C/75% RH for 6 months (top: before storage, bottom: after storage).

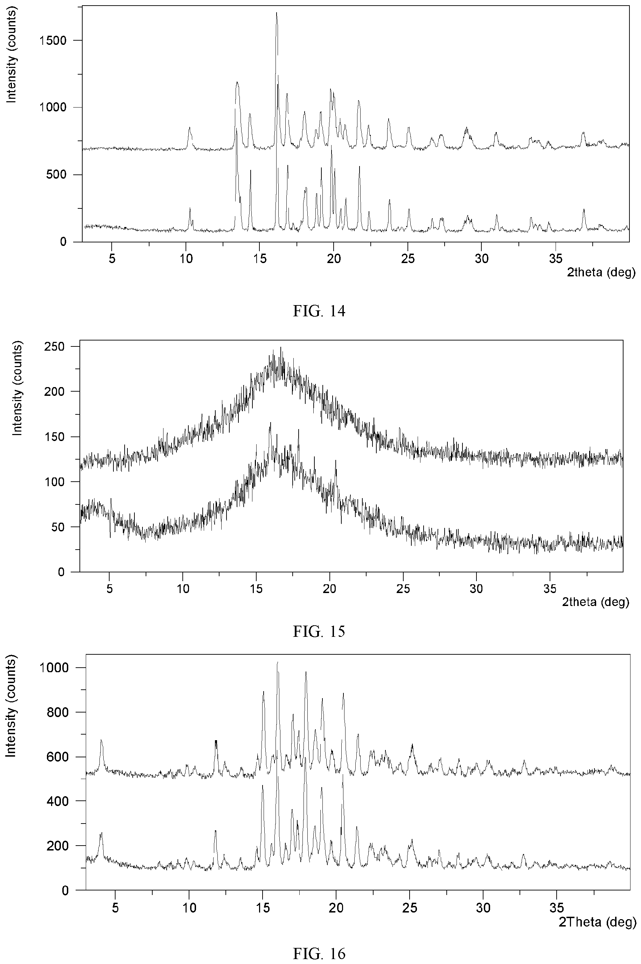

Figure 14 shows an XRPD pattern overlay of Form CS4 before and after stored at 40 °C/75% RH for 6 months (top: before storage, bottom: after storage).
Figure 15 shows an XRPD pattern overlay of the prior art solid before and after stored at 40 °C/75% RH for 6 months (top: before storage, bottom: after storage).
Figure 16 shows an XRPD pattern overlay of Form CS2 before and after stored at 60 °C/75% RH for 2 weeks (top: before storage, bottom: after storage).

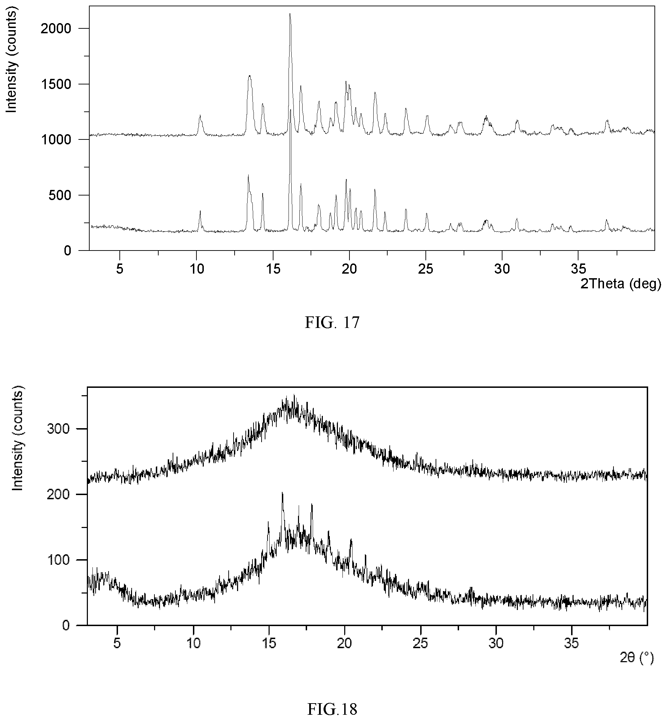

Figure 17 shows an XRPD pattern overlay of Form CS4 before and after stored at 60 °C/75% RH for 2 weeks (top: before storage, bottom: after storage).
Figure 18 shows an XRPD pattern overlay of the prior art solid before and after stored at 60 °C/75% RH for 2 weeks (top: before storage, bottom: after storage).

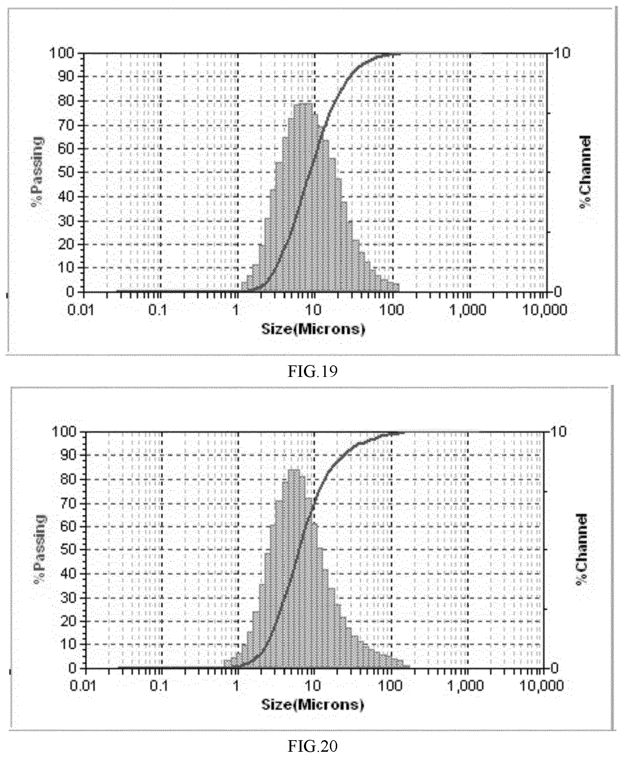

Figure 19 shows a PSD plot of Form CS2.
Figure 20 shows a PSD plot of Form CS4.

### DETAILED DESCRIPTION

The present disclosure is further illustrated by the following examples which describe the preparation and use of the crystalline forms of the present disclosure in detail. It is obvious to those skilled in the art that many changes in the materials and methods can be accomplished without departing from the scope of the present disclosure.

The abbreviations used in the present disclosure are explained as follows
XRPD: X-ray Powder Diffraction
DSC: Differential Scanning Calorimetry
TGA: Thermo Gravimetric Analysis
DVS: Dynamic Vapor Sorption
PSD: Particle Size Distribution

Instruments and methods used for data collection:
X-ray powder diffraction patterns in the present disclosure were acquired by a Bruker D2 PHASER X-ray powder diffractometer. The parameters of the X-ray powder diffraction method of the present disclosure are as follows:
X-ray Reflection: Cu, Kα
Ka1 (Å): 1.54060; Kα2 (Å): 1.54439
Kα2/Kα1 intensity ratio: 0.50
Voltage: 30 (kV)
Current: 10 (mA)
Scan range: from 3.0 degree to 40.0 degree
Differential scanning calorimetry (DSC) data in the present disclosure were acquired by a TA Q2000. The parameters of the DSC method of the present disclosure are as follows:
Heating rate: 10 °C/min unless otherwise specified.
Purge gas: nitrogen
Thermo gravimetric analysis (TGA) data in the present disclosure were acquired by a TA Q500. The parameters of the TGA method of the present disclosure are as follows:
Heating rate: 10 °C/ min
Purge gas: nitrogen
Dynamic Vapor Sorption (DVS) was collected via an SMS (Surface Measurement Systems Ltd.) intrinsic DVS instrument. Its control software is DVS- Intrinsic control software, and its analysis software is DVS-Intrinsic Analysis software. Typical Parameters for DVS test are as follows:
Temperature: 25 °C
Gas and flow rate: N₂, 200 mL/min
dm/dt: 0.002%/min
RH range: 0% RH to 95% RH
Unless otherwise specified, the following examples were conducted at room temperature. Said "room temperature" is not a specific value, and refers to 10-30 °C.

According to the present disclosure, BAY-1002670 used as a raw material is solid (crystalline or amorphous), semisolid, wax or oil. Preferably, compound I used as a raw material is a solid.

Raw materials of BAY-1002670 used in the following examples were prepared by known methods in the prior art, for example, the method disclosed in CN102482317A.

### Example 1-5: Preparation of Form CS2

### Example 1:

1022.4 mg of BAY-1002670 was added into a mixture of water and acetone (4:1, v/v). The mixture was stirred at room temperature for 2 days, and then filtered and dried under vacuum to obtain a solid.

The solid obtained in Example 1 was confirmed to be Form CS2. The XRPD pattern is substantially as depicted in Figure 1 and the XRPD data are listed in Table 1.

**Table 1**

| 2θ | d spacing | Intensity % |
|---|---|---|
| 4.00 | 22.10 | 28.00 |
| 7.94 | 11.14 | 6.02 |
| 8.62 | 10.26 | 2.83 |
| 9.20 | 9.61 | 5.56 |
| 9.76 | 9.07 | 6.80 |
| 10.27 | 8.61 | 5.61 |
| 11.76 | 7.53 | 27.38 |
| 12.29 | 7.20 | 9.38 |
| 13.46 | 6.58 | 6.45 |
| 14.53 | 6.10 | 15.88 |
| 14.95 | 5.93 | 48.82 |
| 15.55 | 5.70 | 20.49 |
| 15.91 | 5.57 | 98.65 |
| 16.51 | 5.37 | 16.36 |
| 16.92 | 5.24 | 34.11 |
| 17.31 | 5.12 | 29.47 |
| 17.87 | 4.96 | 100.00 |
| 18.47 | 4.80 | 26.49 |
| 18.96 | 4.68 | 72.28 |
| 19.59 | 4.53 | 17.53 |
| 20.43 | 4.35 | 66.46 |
| 21.39 | 4.15 | 40.32 |
| 22.16 | 4.01 | 14.70 |
| 23.25 | 3.83 | 18.68 |
| 24.24 | 3.67 | 7.77 |
| 25.06 | 3.55 | 22.49 |
| 26.28 | 3.39 | 8.06 |
| 26.94 | 3.31 | 12.06 |
| 28.86 | 3.09 | 9.18 |
| 29.40 | 3.04 | 6.67 |
| 30.13 | 2.97 | 12.15 |
| 31.91 | 2.81 | 6.07 |
| 32.58 | 2.75 | 8.26 |
| 33.54 | 2.67 | 4.31 |
| 38.46 | 2.34 | 4.93 |

The DSC curve of Form CS2 obtained in Example 1 is substantially as depicted in Figure 2. The first endothermic peak at around 65 °C corresponds to the loss of water, and the second endothermic peak at around 206 °C corresponds to the melting process of Form CS2.

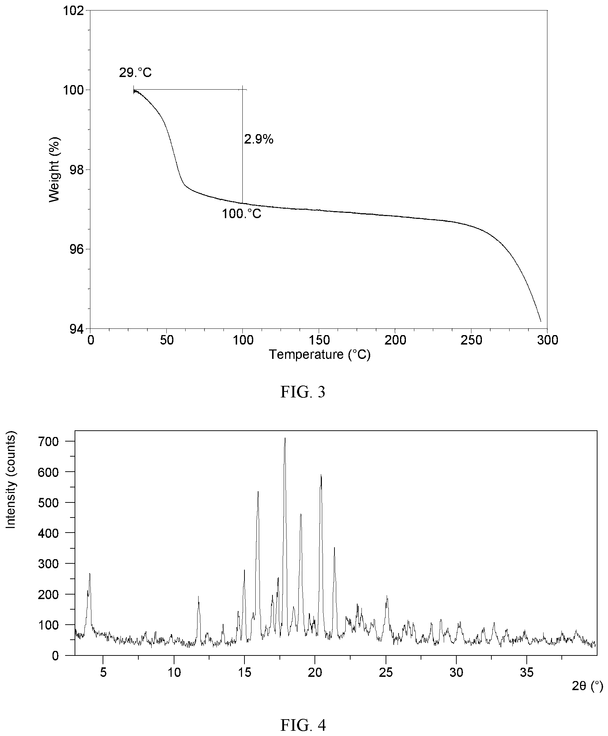

The TGA curve of Form CS2 obtained in Example 1 is substantially as depicted in Figure 3, which shows about 2.9% weight loss when heated to 100 °C.

### Example 2-4:

Certain amount of BAY-1002670 was weighed into glass vials and dissolved in corresponding solvents shown in Table 2. The uncapped glass vials were placed in closed glass containers with anti-solvent shown in table 2. Solids were obtained by liquid vapor diffusion. The solids obtained in Example 2-4 were labeled as samples 2-4.

The solids obtained in Example 2-4 were confirmed to be Form CS2. Sample 2 was selected for characterization, the XRPD pattern is substantially as depicted in Figure 4 and the XRPD data are listed in Table 3.

**Table 2**

| Example | Amount (mg) | Solvent | Volume (mL) | Anti-solvent | Sample |
|---|---|---|---|---|---|
| 2 | 9.5 | butanone | 0.5 | water | Sample 2 |
| 3 | 9.5 | ethyl acetate | 0.5 | water | Sample 3 |
| 4 | 10.9 | methanol | 0.5 | water | Sample 4 |

**Table 3**

| 2θ | d spacing | Intensity % |
|---|---|---|
| 4.07 | 21.69 | 29.08 |
| 7.93 | 11.15 | 3.41 |
| 11.71 | 7.56 | 17.65 |
| 12.34 | 7.18 | 5.15 |
| 13.48 | 6.57 | 7.94 |
| 14.56 | 6.08 | 15.36 |
| 15.02 | 5.90 | 28.13 |
| 15.56 | 5.70 | 13.09 |
| 15.98 | 5.55 | 73.94 |
| 16.98 | 5.22 | 22.11 |
| 17.39 | 5.10 | 32.32 |
| 17.87 | 4.96 | 100.00 |
| 18.50 | 4.80 | 18.08 |
| 19.01 | 4.67 | 62.80 |
| 19.60 | 4.53 | 14.19 |
| 20.49 | 4.34 | 68.77 |
| 21.37 | 4.16 | 46.98 |
| 22.31 | 3.99 | 11.26 |
| 22.98 | 3.87 | 17.31 |
| 23.30 | 3.82 | 15.19 |
| 24.13 | 3.69 | 10.13 |
| 25.09 | 3.55 | 21.79 |
| 26.63 | 3.35 | 10.57 |
| 26.99 | 3.30 | 8.61 |
| 28.25 | 3.16 | 9.67 |
| 28.91 | 3.09 | 12.01 |
| 29.40 | 3.04 | 6.49 |
| 30.22 | 2.96 | 9.03 |
| 31.85 | 2.81 | 6.55 |
| 32.66 | 2.74 | 9.37 |
| 33.58 | 2.67 | 6.02 |
| 34.88 | 2.57 | 4.50 |
| 37.53 | 2.40 | 4.94 |
| 38.50 | 2.34 | 5.86 |

### Example 5:

9.9 mg of BAY-1002670 was weighed into a glass vial and 0.5 mL of methyl isobutyl ketone was added to dissolve BAY-1002670 with stirring. The uncapped glass vials were placed in closed glass containers with n-heptane. The obtained solids were confirmed to be Form CS2. Form CS2 single crystals were obtained in this example. The single crystal X-ray diffraction and structural analysis data are listed in Table 4. The single crystal structure indicates that Form CS2 is a monohydrate.

**Table 4**

| | |
|---|---|
| Molecular formula | C₂₇H₂₉F₅O₄S•H₂O |
| Molecular weight | 562.57 |
| Crystal system | monoclinic |
| Space group | *C2* |
| Unit cell dimensions | *a* = 21.432(2) Å |
| | *b* = 10.7076(10) Å |
| | *c* = 22.438(2) Å |
| | *α* = 90° |
| | *β* = 98.346(3)° |
| | γ = 90° |
| Volume of unit cell: | V = 5094.4(8) Å³ |
| Number of formula units in unit cell | Z = 8 |
| Calculated density | 1.464 g/cm³ |
| X-ray diffractometer | Bruker D8 Venture |
| X-ray source | Model: TURBO X-RAY SOURCE high intensity microfocus rotating anode generator |
| | Wavelength: Mο/Kα (λ = 0.71073) |
| | Power: 2.5 KW |
| Detector | PHOTON 100 model CMOS 2D detector |
| Goniometer | Three-axis (ω, 2θ, ϕ) goniometer |
| Test temperature | 175.15 K |
| Computer program for structure analysis: | Initial structure solution: ShelXT (direct method) |
| | Refinement: ShelXL 2017 (least square method) |

### Example 6-9: Preparation of Form CS4

### Example 6:

102.8 mg of BAY-1002670 was added into 4.0 mL of methyl tert-butyl ether. The mixture was stirred at 50 °C for 18 days. Solid was collected by centrifugation and dried to obtain white crystals.

The obtained white crystals were confirmed to be Form CS4. The XRPD pattern is substantially as depicted in Figure 5 and the XRPD data are listed in Table 5.

The DSC curve of Form CS4 obtained in Example 6 is substantially as depicted in Figure 6. The first endothermic peak at around 217 °C corresponds to the melting process of Form CS4.

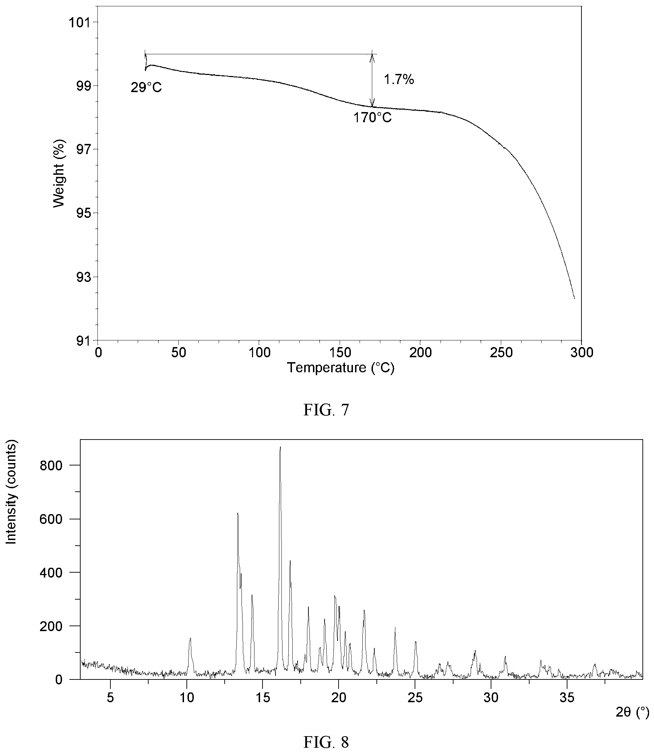

The TGA curve of Form CS4 obtained in Example 6 is substantially as depicted in Figure 7, which shows about 1.7% weight loss when heated to around 170 °C.

**Table 5**

| 2θ | d spacing | Intensity % |
|---|---|---|
| 10.05 | 8.80 | 18.06 |
| 13.20 | 6.71 | 35.97 |
| 13.48 | 6.57 | 22.59 |
| 14.17 | 6.25 | 22.27 |
| 15.95 | 5.56 | 100.00 |
| 16.65 | 5.32 | 41.84 |
| 17.82 | 4.98 | 19.14 |
| 18.59 | 4.77 | 12.72 |
| 18.95 | 4.68 | 26.26 |
| 19.60 | 4.53 | 38.47 |
| 19.84 | 4.47 | 34.38 |
| 20.18 | 4.40 | 16.47 |
| 20.58 | 4.32 | 19.66 |
| 21.48 | 4.14 | 30.48 |
| 22.15 | 4.01 | 18.86 |
| 23.51 | 3.78 | 17.20 |
| 24.87 | 3.58 | 12.33 |
| 26.43 | 3.37 | 5.73 |
| 27.05 | 3.30 | 6.20 |
| 28.83 | 3.10 | 9.90 |
| 30.73 | 2.91 | 7.81 |
| 33.11 | 2.71 | 5.13 |
| 33.71 | 2.66 | 3.90 |
| 34.29 | 2.62 | 3.36 |
| 36.66 | 2.45 | 6.57 |
| 37.83 | 2.38 | 2.37 |

### Example 7:

53.4 mg of BAY-1002670 was dissolved into 2.5 mL of 2-methyltetrahydrofuran. The solution was evaporated at room temperature to obtain a solid. The solid obtained by evaporation was heated to 160 °C at a rate of 10 °C/min and hold at 160 ° C for 10 min to obtain a solid.

The obtained solid was confirmed to be Form CS4. The XRPD pattern is substantially as depicted in Figure 8 and the XRPD data are listed in Table 6.

**Table 6**

| 2θ | d spacing | Intensity% |
|---|---|---|
| 10.04 | 8.81 | 16.52 |
| 13.16 | 6.73 | 72.27 |
| 13.40 | 6.61 | 36.36 |
| 14.09 | 6.29 | 34.81 |
| 15.97 | 5.55 | 100.00 |
| 16.59 | 5.34 | 47.55 |
| 17.80 | 4.98 | 30.66 |
| 18.57 | 4.78 | 12.59 |
| 18.87 | 4.70 | 25.32 |
| 19.54 | 4.54 | 36.21 |
| 19.83 | 4.48 | 25.81 |
| 20.22 | 4.39 | 20.09 |
| 20.50 | 4.33 | 14.51 |
| 21.47 | 4.14 | 28.46 |
| 22.11 | 4.02 | 10.92 |
| 23.49 | 3.79 | 21.95 |
| 24.82 | 3.59 | 15.85 |
| 27.00 | 3.30 | 5.50 |
| 28.76 | 3.10 | 12.04 |
| 30.74 | 2.91 | 8.60 |
| 33.06 | 2.71 | 7.49 |
| 33.67 | 2.66 | 4.48 |
| 34.27 | 2.62 | 2.97 |
| 36.56 | 2.46 | 5.38 |
| 37.84 | 2.38 | 2.59 |

### Example 8-9:

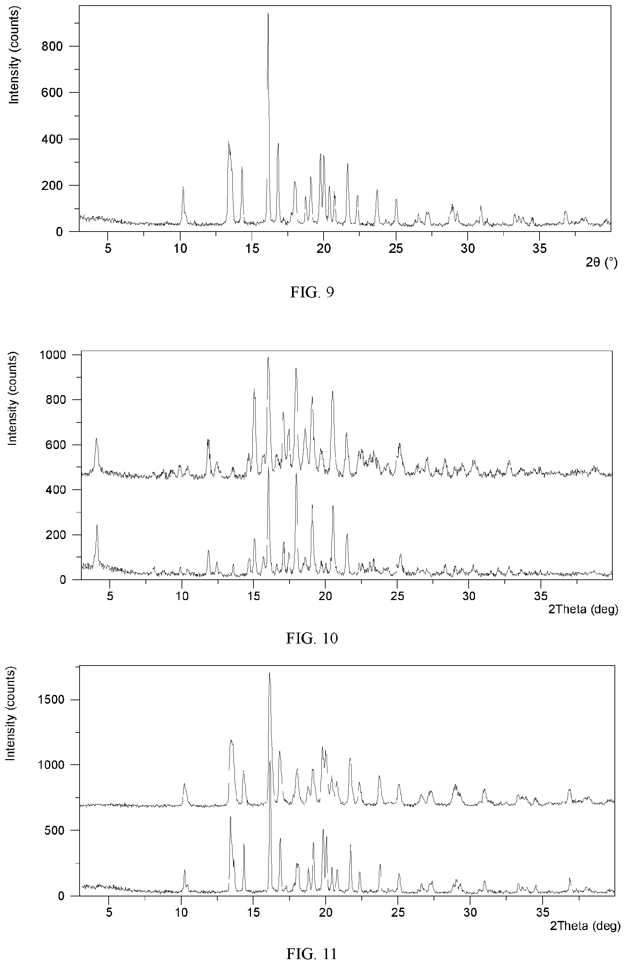

Certain amount of BAY-1002670 was weighed into glass vials, and the uncapped glass vials were placed in closed glass containers with anti-solvent shown in table 7. The solids were obtained by solid vapor diffusion. The solids obtained were confirmed to be Form CS4. Sample 8 was selected for characterization. The XRPD pattern is substantially as depicted in Figure 9 and the XRPD data are listed in Table 8.

**Table 7**

| Example | Amount (mg) | Solvent | Volume (mL) | Sample |
|---|---|---|---|---|
| 8 | 7.5 | Ethanol | 5.0 | 8 |
| 9 | 6.2 | Isopropyl acetate | 5.0 | 9 |

**Table 8**

| 2θ | d Spacing | Intensity% |
|---|---|---|
| 10.08 | 8.78 | 18.80 |
| 13.23 | 6.69 | 37.24 |
| 13.47 | 6.57 | 25.59 |
| 14.17 | 6.25 | 27.35 |
| 15.97 | 5.55 | 100.00 |
| 16.67 | 5.32 | 34.80 |
| 17.80 | 4.98 | 17.22 |
| 18.61 | 4.77 | 13.34 |
| 18.94 | 4.69 | 22.74 |
| 19.63 | 4.52 | 33.62 |
| 19.87 | 4.47 | 31.33 |
| 20.25 | 4.39 | 18.05 |
| 20.61 | 4.31 | 12.96 |
| 21.53 | 4.13 | 28.55 |
| 22.20 | 4.00 | 14.07 |
| 23.58 | 3.77 | 16.21 |
| 24.88 | 3.58 | 12.52 |
| 26.46 | 3.37 | 4.72 |
| 26.98 | 3.31 | 5.54 |
| 27.18 | 3.28 | 4.96 |
| 28.85 | 3.09 | 8.24 |
| 30.77 | 2.91 | 8.11 |
| 33.12 | 2.70 | 5.30 |
| 33.71 | 2.66 | 3.75 |
| 34.34 | 2.61 | 2.81 |
| 36.65 | 2.45 | 6.02 |
| 38.07 | 2.36 | 3.02 |

### Example 10: Hygroscopicity of Form CS2, Form CS4 and the prior art amorphous

Dynamic vapor sorption (DVS) was applied to test hygroscopicity of Form CS2, Form CS4 and the prior art amorphous with about 10 mg of samples. The results are listed in Table 9.

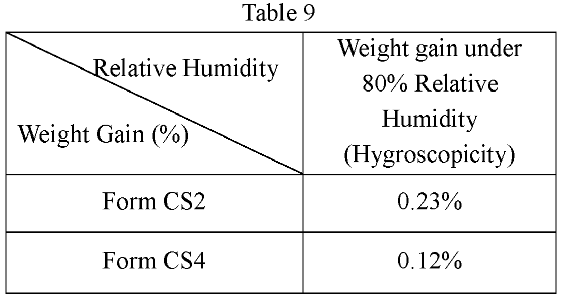

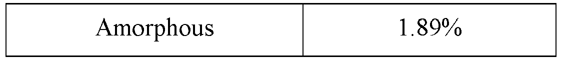

The result shows that Form CS2 and Form CS4 of the present disclosure have lower hygroscopicity compared with the prior art amorphous.

### Example 11: Stability of Form CS2, Form CS4 and the prior art amorphous

### 1. Physical and chemical stability under condition of 25 °C/60% RH

Samples of Form CS2, Form CS4 and the prior art amorphous were stored under condition of 25 °C/60% RH (open). Crystalline form and chemical impurity were tested. The results are shown in Table 10 and Table 11.

**Table 10**

| Initial Solid Form | Storage Condition | Time | Solid Form after Storage |
|---|---|---|---|
| Form CS2 (Figure 10, top) | 25 °C/60% RH | 6 months | Form CS2 (Figure 10, bottom) |
| Form CS4 (Figure 11, top) | 25 °C/60% RH | 6 months | Form CS4 (Figure 11, bottom) |
| Amorphous (Figure 12, top) | 25 °C/60% RH | 6 months | Amorphous (Figure 12, bottom) |

**Table 11**

| Solid Form | Initial Purity | Purity after 6 Months | Change of Purity |
|---|---|---|---|
| CS2 | 98.64% | 98.58% | 0.06% |
| CS4 | 99.44% | 99.31% | 0.13% |
| Amorphous | 98.68% | 97.88% | 0.80% |

The crystalline form of Form CS2 and Form CS4 of the present disclosure remained unchanged for at least 6 months when stored under the condition of 25 °C/60% RH. The purity remained substantially unchanged during storage. The reduction in purity of Form CS2 and Form CS4 was only 0.06% and 0.13%, respectively, while the purity of amorphous was reduced by 0.80%.

The above results indicate that Form CS2 and Form CS4 of the present disclosure have good physical and chemical stability, and the purity remained substantially unchanged during drug storage, Form CS2 and CS4 are more suitable for medicinal use. While the impurity of the prior art amorphous increased, which will lead to significantly lowered active ingredient content or reduced drug activity, and will also lead to significantly increased toxicity and side effects of the drug products.

### 2. Physical and chemical stability under accelerated condition of 40 °C/75% RH

Samples of Form CS2, Form CS4 and the prior art amorphous were stored under condition of 40 °C/75% RH (open). Crystalline form and chemical impurity were tested. The results are shown in Table 12 and Table 13.

**Table 12**

| Initial Solid Form | Storage Condition | Time | Solid Form after Storage |
|---|---|---|---|
| Form CS2 (Figure 13, top) | 40 °C/75% RH | 6 months | Form CS2 (Figure 13, bottom) |
| Form CS4 (Figure 14, top) | 40 °C/75% RH | 6 months | Form CS4 (Figure 14, bottom) |
| Amorphous (Figure 15, top) | 40 °C/75% RH | 6 months | Amorphous + Form CS2 (Figure 15, bottom) |

**Table 13**

| Solid Form | Initial Purity | Purity after 3 Months | Change of Purity |
|---|---|---|---|
| CS2 | 98.64% | 98.37% | 0.27% |
| CS4 | 99.44% | 99.42% | 0.02% |
| Amorphous | 98.68% | 97.60% | 1.08% |

The crystalline form of Form CS2 and Form CS4 of the present disclosure remained unchanged for at least 6 months when stored under the condition of 40 °C/75% RH, while the prior art amorphous partially converted to Form CS2 after stored under the condition of 40 °C/75% RH for 6 months. The purity of Form CS2 and Form CS4 of the present disclosure remained substantially unchanged when stored under the condition of 40 °C/75% RH. The reduction in purity of Form CS2 and Form CS4 was only 0.27% and 0.02%, respectively, while the purity of amorphous was reduced by 1.08%. The above results indicate that, compared with the amorphous of the prior art, Form CS2 and Form CS4 of the present disclosure have good physical and chemical stability.

### 3. Physical and chemical stability under accelerated condition of 60 °C/75% RH

Samples of Form CS2, Form CS4 and the prior art amorphous were stored under condition of 60 °C/75% RH (open). Crystalline form and chemical impurity were tested. The results are shown in Table 14 and Table 15.

**Table 14**

| Initial Solid Form | Storage Condition | Time | Solid Form after Storage |
|---|---|---|---|
| Form CS2 (Figure 16, top) | 60 °C/75% RH | 2 weeks | Form CS2 (Figure 16, bottom) |
| Form CS4 (Figure 17, top) | 60 °C/75% RH | 2 weeks | Form CS4 (Figure 17, bottom) |
| Amorphous (Figure 18, top) | 60 °C/75% RH | 2 weeks | Amorphous + Form CS2 (Figure 18, bottom) |

**Table 15**

| Solid Form | Initial Purity | Purity after 2 Weeks | Change of Purity |
|---|---|---|---|
| CS2 | 98.64% | 98.59% | 0.05% |
| CS4 | 99.44% | 99.47% | 0.03% |
| Amorphous | 98.68% | 97.70% | 0.98% |

The crystalline form of Form CS2 and Form CS4 of the present disclosure remained unchanged for at least 2 weeks when stored under the condition of 60 °C/75% RH, while the prior art amorphous partially converted to Form CS2 after stored under the condition of 40 °C/75% RH for 2 weeks. The purity of Form CS2 and Form CS4 of the present disclosure remained substantially unchanged when stored under the condition of 60 °C/75% RH. The reduction in purity of Form CS2 and Form CS4 was only 0.05% and 0.03%, respectively, while the purity of amorphous was reduced by 0.98%. The above results indicate that, compared with the amorphous of the prior art, Form CS2 and Form CS4 of the present disclosure have good physical and chemical stability.

### Example 12: Residual solvents of Form CS2, Form CS4 and the prior art amorphous

Residual solvents of Form CS2, Form CS4 and the prior art amorphous were tested. The results show that Form CS2 and Form CS4 have almost no residual solvents, while the dichloromethane residue in the amorphous solid is 64185 ppm. According to the guideline of the International Council for Harmonization (ICH) on residual solvents, dichloromethane belongs to Class 2 solvents, and the residual solvent content must not exceed 600 ppm. It can be seen that the residue of dichloromethane in the amorphous solid is much higher than the limits of ICH guideline, and the amorphous not suitable for use as drug substance.

### Example 13: Particle size distribution

Form CS2 and Form CS4 of the present disclosure were added into 10 mL of Isopar G (containing 0.2% lecithin). The mixture was mixed thoroughly and transferred into the SDC. The measurement was started when the sample amount indicator is in appropriate position. The average particle diameter calculated by volume, the diameter at which 10% mass is comprised of smaller particles, the diameter at which 50% mass is comprised of smaller particles and the diameter at which 90% mass is comprised of smaller particles were obtained in particle size distribution test. Subsequently, the particle size distribution plot of crystalline forms was obtained. The results are shown in Table 16.

**Table 16**

| Solid Form | MV(µm) | D10 (µm) | D50 (µm) | D90 (µm) |
|---|---|---|---|---|
| Form CS2 | 13.16 | 3.09 | 8.41 | 27.45 |
| Form CS4 | 11.41 | 2.32 | 6.20 | 23.12 |

| | | | | |
|---|---|---|---|---|
| Notes: MV: Average particle diameter calculated by volume. D10: the size in microns below which 10 percent of the particles reside on a volume basis. D50: the size in microns below which 50 percent of the particles reside on a volume basis, also known as the median diameter. D90: the size in microns below which 90 percent of the particles reside on a volume basis. The particle size distribution (PSD) plot of Form CS2 and Form CS4 are shown in Figure 19 and Figure 20, respectively. It can be seen that the particle size of Form CS2 and Form CS4 is in monodisperse normal distribution. The particle size is uniform and small. | | | | |

Form CS2 and Form CS4 of the present disclosure have uniform and small particle size, which is helpful to simplify the post-treatment of the formulation process, for example, less grinding can save cost. Additionally, the uniform particle size distribution of Form CS2 and Form CS4 improves the uniformity of drug substance in drug products; smaller particle size distribution can increase the specific surface area of the drug substance, and improve the dissolution rate of the drug, thereby facilitating drug absorption and further improving bioavailability of the drug.

The examples described above are only for illustrating the technical concepts and features of the present disclosure, and intended to make those skilled in the art being able to understand the present disclosure and thereby implement it, and should not be concluded to limit the protective scope of this disclosure. Any equivalent variations or modifications according to the spirit of the present disclosure should be covered by the protective scope of the present disclosure.

## Claims

1. A crystalline form CS2 of BAY-1002670, wherein the X-ray powder diffraction pattern shows characteristic peaks at 2theta values of 4.0°±0.2°, 15.9°±0.2° and 17.9°±0.2° using CuKa radiation.

2. The crystalline form CS2 according to claim 1, wherein the X-ray powder diffraction pattern shows one or two or three characteristic peaks at 2theta values of 19.0°±0.2°, 20.4°±0.2° and 21.4°±0.2°.

3. The crystalline form CS2 according to claim 1, wherein the X-ray powder diffraction pattern shows one or two or three characteristic peaks at 2theta values of 11.8°±0.2°, 15.0°±0.2° and 25.1°±0.2°.

4. The crystalline form CS2 according to any one of claims 1 to 3, wherein the crystalline form CS2 belongs to monoclinic system, the space group of crystalline form CS2 is *C2,* and the crystal axes are: *a*=21.432(2) Å, *b*=10.7076(10) Å, *c*=22.438(2) Å, the interaxial angles are: *α* = 90 °, *β* = 98.346(3) *°, γ* = 90 °.

5. A process for preparing crystalline form CS2 according to claim 1, wherein the process comprises:
(1) adding BAY-1002670 into a mixture of ketones and water, and stirring to obtain crystalline form CS2 ; or
(2) dissolving BAY-1002670 in alcohols, esters, or ketones, putting the solution in a closed system with water vapor to obtain crystalline form CS2 by liquid vapor diffusion; or
(3) dissolving BAY-1002670 in ketones, putting the solution in a closed system with n-heptane vapor to obtain crystalline form CS2 by liquid vapor diffusion.

6. The process according to claim 5, wherein in method (1), said ketone is acetone; in method (2), said alcohol is methanol, said ester is ethyl acetate, said ketone is butanone; in method (3), said ketone is methyl isobutyl ketone.

7. A crystalline form CS4 of BAY-1002670, wherein the X-ray powder diffraction pattern shows characteristic peaks at 2theta values of 16.0°±0.2°, 16.6°±0.2° and 14.1°±0.2° using CuKa radiation.

8. The crystalline form CS4 according to claim 7, wherein the X-ray powder diffraction pattern shows one or two or three characteristic peaks at 2theta values of 18.9°±0.2°, 21.5°±0.2° and 10.1°±0.2°.

9. The crystalline form CS4 according to claim 7, wherein the X-ray powder diffraction pattern shows one or two or three characteristic peaks at 2theta values of 13.2°±0.2°, 23.5°±0.2° and 17.9°±0.2°.

10. A process for preparing crystalline form CS4 according to claim 7, wherein the process comprises:
(1) adding BAY-1002670 into ethers, and stirring at 40 °C - 60 °C to obtain crystalline form CS4; or
(2) dissolving BAY-1002670 in 2-methyltetrahydrofuran, evaporating to obtain a solid, and heating the solid to obtain crystalline form CS4; or
(3) adding BAY-1002670 in a closed system with alcohol or ester vapor to obtain crystalline form CS4 by solid vapor diffusion.

11. The process for preparing crystalline form CS4 according to claim 10, wherein in method (1), said ether is methyl tert-butyl ether, said stirring temperature is 50 °C; in method (2), said heating temperature is 160 °C; in method (3), said alcohol is ethanol, said ester is isopropyl acetate.

12. A pharmaceutical composition, wherein said pharmaceutical composition comprises a therapeutically effective amount of crystalline form CS2 according to claim 1 or crystalline form CS4 according to claim 7, or combinations thereof and pharmaceutically acceptable carriers, diluents or excipients.

13. Crystalline form CS2 according to claim 1 or crystalline form CS4 according to claim 7, or combinations thereof for the use of preparing selective progesterone receptor modulator drugs.

14. Crystalline form CS2 according to claim 1 or crystalline form CS4 according to claim 7, or combinations thereof for the use of preparing drugs treating uterine fibroids and/or endometriosis.
